# EUROPEAN PATENT SPECIFICATION

(11) **EP 1 333 889 B1**
(45) Date of publication and mention of the grant of the patent: **07.03.2007**
(21) Application number: 01986486.7
(22) Date of filing: 14.11.2001
(51) Int. Cl.: A62D 1/00

(54) **FIRE EXTINGUISHING METHODS UTILIZING HYDROFLUOROETHERS**
FEUERLÖSCHVERFAHREN UNTER VERWENDUNG VON HYDROFLUOROETHERN
PROCEDES D'EXTINCTION UTILISANT DES HYDROFLUOROETHERS

(30) Priority: 17.11.2000 US 249684 P
(43) Date of publication of application: 13.08.2003
(73) Proprietor: GREAT LAKES CHEMICAL CORPORATION, West Lafayette, IN 47906 (US)
(72) Inventor: ROBIN, Mark, L., West Lafayette, IN 47906 (US); Rowland, Thomas, F., El Dorado, AR 71730 (US)
(74) Representative: Hackett, Sean James
(86) International application number: PCT/US2001/044256
(87) International publication number: WO 2002/040102

(56) References cited:
- EP-A- 0 562 858
- WO-A-01/05468
- WO-A-93/24586
- WO-A-96/40834
- US-A- 2 730 543
- US-A- 3 557 294
- US-A- 3 943 256
- US-A- 5 562 861
- US-A- 5 730 894
- US-A- 6 023 002
- FUKAYA H ET AL: "Fire extinguishing ability of perfluoroalkylamines and perfluoroethers evaluated by a small cup burner method" JOURNAL OF FLUORINE CHEMISTRY, ELSEVIER SEQUOIA, LAUSANNE, CH, vol. 106, no. 2, December 2000 (2000-12), pages 143-146, XP004219156 ISSN: 0022-1139
- PATENT ABSTRACTS OF JAPAN vol. 1995, no. 04, 31 May 1995 (1995-05-31) & JP 07 025803 A (AGENCY OF IND SCIENCE & TECHNOL;OTHERS: 04), 27 January 1995 (1995-01-27)

## Description

### FIELD OF THE INVENTION

The present invention is directed to hydrofluoroether fire extinguishing agents and methods for extinguishing fires using the hydrofluoroethers. More particularly, the present invention is directed to fire extinguishing agents and methods using saturated, fluorinated C₄ and/or C₃ hydrofuoroethers, and blends of one or more of the hydrofluoroethers with one or more other fire extinguishing agents.

### BACKGROUND OF THE INVENTION AND PRIOR ART

The use of certain bromine, chlorine and iodine-containing halogenated chemical agents for the extinguishment of fires is common. These agents are in general thought to be effective due to their interference with the normal chain reactions responsible for flame propagation. The most widely accepted mechanism for flame suppression is the radical trap mechanism proposed by Fryburg in *Review of Literature Pertinent to Fire Extinguishing Agents and to Basic Mechanisms Involved in Their Action,* NACA-TN 2102 (1950). The finding that the effectiveness of the halogens are on a molar basis in the order Cl < Br < I supports the radical trap mechanism, as reported by Malcom in *Vaporizing Fire Extinguishing Agents,* Report 117, Dept. of Army Engineering Research and Development Laboratories, Fort Bevoir, VA, 1950 (Project- 8-76-04-003). It is thus generally accepted that compounds containing the halogens Cl, Br and I act by interfering with free radical or ionic species in the flame and that the effectiveness of these halogens is in the order I > Br > Cl. In addition, it is generally thought that to be effective as a fire extinguishing agent, a compound must contain Cl, Br or I.

The use of iodine-containing compounds as fire extinguishing agents has been avoided primarily due to the expense of their manufacture or due to toxicity considerations. Until very recently, the three fire extinguishing agents presently in common use were all bromine-containing compounds, Halon 1301 (CF₃Br), Halon 1211 (CF₂BrCl) and Halon 2402 (BrCF₂CF₂Br). The effectiveness of these three volatile bromine-containing compounds in extinguishing fires has been described in U.S. Pat. No. 4,014,799 to Owens. Although not employed commercially, certain chlorine-containing compounds are also known to be effective extinguishing agents, for example Halon 251 (CF₃CF₂Cl) as described by Larsen in U.S. Pat. No. 3,844,354.

Although the above named bromine or chlorine-containing Halons are effective fire fighting agents, those agents containing bromine or chlorine are asserted by some to be capable of the destruction of the earth's protective ozone layer. Also, because the agents contain no hydrogen atoms which would permit their destruction in the troposphere, the agents may also contribute to the greenhouse warming effect.

WO93/24586 discloses the compounds (CF₃)₂C=CFOCH₃ and CF₃CF=CFOCH₃, used as fire extinguishing agents. They may be used alone or in combination with other chemicals.

More recently, hydrofluorocarbons have been proposed as fire suppression, for example in U.S. Pat. No. 5,124,053. However, a disadvantage of these compounds is their relatively high global warming potential.

It is therefore an object of this invention to provide a method for extinguishing fires that extinguishes fires as rapidly and effectively as the techniques employing Halon agents while avoiding the above-named drawbacks.

It is a further object of this invention to provide an agent for the use in a method of the character described that is efficient, economical to manufacture, and environmentally safe with regard to ozone depletion and greenhouse warming effects.

It is a still further object of this invention to provide blends of the new agents and other fire extinguishing agents that are effective and environmentally safe.

### SUMMARY OF THE INVENTION

The foregoing and other objects, advantages and features of the present invention may be achieved by employing saturated, higher fluorinated hydrofluoroethers and blends thereof with other agents as fire extinguishants for use in fire extinguishing methods and apparatus. More particularly, the method of this invention involves introducing to a fire a saturated, fluorinated C₄ or C₅ hydrofluoroether in a fire extinguishing concentration selected from the group consisting of:
CF₃CHFCF₂OCH₃, CF₃CHFCF₂OCH₂F, CF₃CHFCF₂OCF₂H, CF₃CHFCF₂OCF₃, (CF₃)₂CHCF₂OCH₃, (CF₃)₂CHCF₂OCH₂F. (CF)₂CHCF₂OCHF₂ and (CF₃)₂CHCF₂0CF₃.

These hydrofluoroethers may be used alone, in admixture with each other or as blends with other fire extinguishing agents. Generally, the agents of this invention are employed at concentrations lying in the range of about 3 to 15%, preferably 5 to 10% in air, on a v/v basis . The agents of this invention are suitable for use in both total flooding and portable fire suppression applications. Suitable extinguishing agents ('blends') for admixture with the hydrofluoroethers include CF₃CHFCF₃, CF₃CF₂CF₂H, CF₃CH₂CF₃, CF₃CF₂H, and CF₃H.

The hydrofluoroethers of this invention may be produced via numerous routes. For example, CF₃CEFCF₂OCF₂H may be prepared via a three step process comprising
(i) reaction of methanol with commercially available hexafluoropropene (CF₃CF=CF₂) in the presence of base to produce CF₃CHFCF₂OCH₃;
(ii) chlorination of CF₃CHFCF₂OCH₃ with Cl₂ to produce CF₃CHFCF₂OCHCl₂; and
(iii) fluorination of CF₃CHFCF₂OCHCl₂ with HF to produce the final product CF₃CHFCF₂OCF₂H.

By further reacting with a strong base like sodium or potassium hydroxide the corresponding unsatured C₄ or C₅ hydrofluoroethers may be prepared.

### DESCRIPTION OF THE PREFERRED EMBODIMENTS

In accordance with the present invention, saturated C₄ and C₅ hydrofluoroethers have been found to be effective fire extinguishants at concentrations safe for use. However, because such hydrofluoroethers contain no bromine or chlorine, they have an ozone depletion potential of zero. Furthermore, since the compounds are characterized by short atmospheric lifetimes they are susceptible to breakdown in the lower atmosphere and hence do not pose a threat as greenhouse warming gasses.

Specific hydrofluoroethers useful in accordance with this invention are:
CF₃CHFCF₂OCH₃, CF₃CHFCF₂OCH₂F, CF₃CHFCF₂OCF₂H, CF₃CHFCF₂OCF₃, (CF₃)₂CHCF₂OCH₃, (CF₃)₂CHCF₂OCH₂F, (CF₃)₂CHCF₂OCHF₂, (CF₃)₂CHCF₂OCF₃.

These hydrofluoroethers may be used alone, in admixture with each other or as blends with other fire extinguishing agents. Generally, when a single hydrofluoroether of this invention is employed, concentrations lying in the range of about 3 to 15%, preferably 5 to 10 % in air, on a v/v basis, are used; when employed in admixture, concentrations lying in the range of about 3 to 15 % , preferably 5 to 10% in air, on a v/v basis, are used. Where the hydrofluoroethers of this invention are employed in admixture with other fire extinguishing agents ('blends'), the hydrofluoroethers desirably comprise of at least about 10 percent by weight of the blend, and the overall concentration of the blend lies in the range of about 3 to 15%, preferably 5 to 10 % in air, on a v/v basis. The agents of this invention are suitable for use in both total flooding and portable fire suppression applications. Suitable extinguishing agents for admixture with the hydrofluoroethers include CF₃CHFCF₃, CF₃CF₂CF₂H, CF₃CH₂CF₃, CF₃CF₂H, and CF₃H.

The C₄ or C₅ hydrofluoroethers of this invention may be effectively employed at substantially any minimum concentrations at which fire may be extinguished, the exact minimum level being dependent on the particular combustible material, the particular hydrofluoroether and the combustion conditions. In general, however, best results are achieved where the hydrofluoroethers or mixtures and blends thereof are employed at a level of at least about 3% (v/v). Where hydrofluoroethers alone are employed, best results are achieved with agent levels of at least about 5 % (v/v). Likewise, the maximum amount to be employed will be governed by matters of economics and potential toxicity to living things. About 15 % (v/v) provides a convenient maximum concentration for use of hydrofluoroethers and mixtures and blends thereof in occupied areas. Concentrations above 15 % (v/v) may be employed in unoccupied areas, with the exact level being determined by the particular combustible material, the hydrofluoroether (or mixture or blend thereof) chosen and the conditions of combustion. The preferred concentration of the hydrofluoroether agents, mixtures and blends in accordance with this invention lies in the range of about 5 to 10% (v/v).

Hydrofluoroethers may be applied using conventional application techniques and methods used for Halons such as Halon 1301 and Halon 1211. Thus, these agents may be used in a total flooding fire extinguishing system in which the agent is introduced to an enclosed region (e.g., a room or other enclosure) surrounding a fire at a concentration sufficient to extinguish the fire. In accordance with a total flooding system apparatus, equipment or even rooms or enclosures may be provided with a source of agent and appropriate piping, valves, and controls so as automatically and/or manually to be introduced an appropriate concentrations in the event that fire should break out. Thus, as is known to those skilled in the art, the fire extinguishant may be pressurized with nitrogen or other inert gas at up to about 600 psig at ambient conditions.

Alternatively, the hydrofluoroether agents may be applied to a fire through the use of conventional portable fire extinguishing equipment. It is usual to increase the pressure in portable fire extinguishers with nitrogen or other inert gasses in order to insure that the agent is completely expelled from the extinguisher. Hydrofluoroether containing systems in accordance with this invention may be conveniently pressurized at any desirable pressure up to about 600 psig at ambient conditions.

The compounds of the present invention are nondestructive agents, and are especially useful where cleanup of other media poses a problem. Some of the applications of the hydrofluoroethers of this invention are the extinguishing of liquid and gaseous fueled fires, the protection of electrical equipment, ordinary combustibles such as wood, paper and textiles, hazardous solids, and the protection of computer facilities, data processing equipment and control rooms.

The invention will be further described with reference to the following specific Examples. However it will be understood that these Examples are illustrative in nature and not restrictive in nature.

### EXAMPLE 1

This example demonstrates the desirable "throw" obtainable with the fire suppression agents of the present invention when employed in portable ("streaming") applications. The throw is the distance the stream of agent can be discharged; the longer the throw the better, as this allows extinguishment without approaching the fire at too close a distance, which can lead to exposure of the operator to fire and toxic fumes from the combustion process.

A 150 mL SS cylinder was equipped with an inlet tube and a dip tube connected via an on/off valve to a delivery nozzle. The cylinder was charged with 50 grams of CF₃CHFCF₂OCF₂H and then pressurized with nitrogen to the desired pressure. The cylinder contents were completely discharged and the throw distance noted (Table 1).

**TABLE 1**

| **Throw vs. Pressure for CF₃CHFCF₂OCF₂H System** | |
|---|---|
| **Pressure, psig** | **Throw, feet** |
| 25 | 10 |
| 80 | 15 |
| 120 | 17 |
| 150 | 18 |

### EXAMPLE 2

This example demonstrates the extinguishment of Class B fires with the agents of the present invention. A 150 mL SS cylinder was equipped with an inlet tube and a dip tube connected via an on/off valve to a delivery nozzle. The cylinder was charged with 30 grams of CF₃CHFCF₂OCF₂H and then pressurized with nitrogen to 120 psig. A 2 inch x 4 inch x 0.5 inch SS pan was filled with 20 mL of methanol. The methanol was ignited and allowed to burn for 30 seconds; the agent was then discharged from a distance of 4 feet onto the fire. The methanol fire was extinguished in 1.5 seconds; a total of 16 grams of agent was discharged.

### EXAMPLE 3

The method of Example 2 was employed with acetone, isopropanol and heptane fuels. All fires were rapidly extinguished (see Table 2).

**TABLE 2**

| **Extinguishment with CF₃CBFCF₂OCF₂H** | | |
|---|---|---|
| **Fuel** | **Extinguishing Time, seconds** | **Agent discharged, grams** |
| acetone | 2.0 | 25 |
| isopropanol | 1.5 | 21 |
| heptane | 1.8 | 11 |

### EXAMPLE 4

This example demonstrates the extinguishment of deep-seated Class A fires with the agents of the present invention. A 150 mL SS cylinder was equipped with an inlet tube and a dip tube connected via an on/off valve to a delivery nozzle. The cylinder was charged with 30 grams of CF₃CHFCF₂OCF₂H and then pressurized with nitrogen to 120 psig. A wood crib was constructed of six layers of 6 inch x 2 inch by 0.125 inch strips of kiln dried fir, each layer consisting of 4 pieces. The crib was soaked with heptane, ignited, and allowed to burn for five minutes. The agent was then discharged onto the fire, resulting in rapid (< 2 seconds) extinguishment; a total of 25 grams of agent was discharged. Immediately after extinguishment the wood crib was cold to the touch, demonstrating the efficient suppression afforded by the agent.

## Claims

1. A method for extinguishing a fire comprising the steps of introducing to the fire a fire extinguishing concentration of a composition comprising a compound selected from the group consisting of CF₃CHFCF₂OCH₃, CF₃CHFCF₂OCH₂F, CF₃CHFCF₂0CF₂H, CF₃CHFCF₂OCF₃, (CF₃)₂CHCF₂OCH₃, (CF₃)₂CHCF₂0CH₂F, (CF₃)₂CHCF₂OCHF₂, and (CF₃)₂CHCF₂OCF₃ and maintaining the concentration of the compound until the fire is extinguished.

2. The method of claim 1, wherein the compound is employed at a level of at least about 3% (v/v).

3. The method of claim 1, wherein the compound is employed in a total flooding system.

4. The method of claim 1, wherein the compound is employed in a portable extinguishing system.

5. The method of claim 1, wherein the composition comprises a blend with other fire extinguishing agents.

6. The method of claim 5, wherein the other fire extinguishing agents are selected from the group consisting of CF₃CHFCF₃, CF₃CF₂CF₂H, CF₃CH₂CF₃, CF₃CF₂H, and CF₃H.

7. A fire extinguishing agent comprising a compound selected from the group consisting of CF₃CHFCF₂OCH₃, CF₃CHFCF₂OCH₂F, CF₃CHFCF₂OCF₂H, CF₃CHFCF₂OCF₃, (CF₃)₂CHCF₂OCH₃, (CF₃)₂CHCF₂OCH₂F, (CF₃)₂CHCF₂OCHF₂, (CF₃)₂CHCF₂OCF₃, and CF₃CF=CFOCH₃.

8. A method of making CF₃CHFCF₂OCF₂H comprising the steps of:
reacting methanol with hexafluoropropene in the presence of base to produce CF₃CHFCF₂OCH₃;
chlorinating CF₃CHFCF₂OCH₃ with Cl₂ to produce CF₃CHFCF₂OCHCl₂; and
fluorinating CF₃CHFCF₂OCHCl₂ with HF to produce CF₃CHFCF₂OCF₂H.

9. A method of making a saturated, fluorinated C₄ or C₅ hydrofluoroether selected from the group consisting of CF₃CHFCF₂OCH₃, CF₃CHFCF₂OCH₂F, CF₃CHFCF₂OCF₂H, CF₃CHFCF₂OCF₃, (CF₃)₂CHCF₂OCH₃, (CF₃)₂CHCF₂OCH₂F, (CF₃)₂CHCF₂OCHF₂, and (CF₃)₂CHCF₂OCF₃, comprising the steps of:
reacting a C1 alcohol with a fluorinated C₃ or C₄ alkene in the presence of a base to form a first reaction product;
chlorinating the first reaction product with Cl₂ to form a second reaction product; and
fluorinating the second reaction product with HF to form a saturated, fluorinated C₄ or C₅ hydrofluoroether.

10. The method of claim 9, wherein the base is selected from the group consisting of sodium and potassium hydroxide.

11. The composition comprising CF₃CHFCF₂OCF₃.

## Patentansprüche

1. Verfahren zum Feuerlöschen, umfassend die Schritte des Einführens einer feuerlöschenden Konzentration einer Zusammensetzung in das Feuer, wobei die Zusammensetzung eine Verbindung aufweist, die ausgewählt ist aus der Gruppe von CF₃CHFCF₂OCH₃, CF₃CHFCF₂OCH₂F, CF₃CHFCF₂OCF₂H, CF₃CHFCF₂OCF₃, (CF3)₂CHCF₂OCH₃, (CF₃)₂CHCF₂OCH₂F, (CF₃)₂CHCF₂OCHF₂ und (CF₃)₂CHCF₂OCF₃, und Aufrechterhalten der Konzentration der Verbindung, bis das Feuer gelöscht ist.

2. Verfahren nach Anspruch 1, wobei die Verbindung in einer Menge von mindestens etwa 3% (Volumen/Volumen) eingesetzt wird.

3. Verfahren nach Anspruch 1, bei welchem die Verbindung in einem System der Totalflutung eingesetzt wird.

4. Verfahren nach Anspruch 1, wobei die Verbindung in einem tragbaren Löschsystem eingesetzt wird.

5. Verfahren nach Anspruch 1, wobei die Zusammensetzung ein Gemisch mit anderen Feuerlöschmitteln aufweist

6. Verfahren nach Anspruch 5, wobei die anderen Feuerlöschmittel ausgewählt sind aus der Gruppe, bestehend aus CF₃CHFCF₃, CF₃CF₂CF₂H, CF₃CH₂CF₃, CF₃CF₂H und CF₃H.

7. Feuerlöschmittel, aufweisend eine Verbindung, die ausgewählt ist aus der Gruppe, bestehend aus: CF₃CHFCF₂OCH₃, CF₃CHFCF₂OCH₂F, CF₃CHFCF₂OCF₂H, CF₃CHFCF₂OCF₃, (CF₃)₂CHCF₂OCH₃, (CF₃)₂CHCF₂OCH₂F, (CF₃)₂CHCF₂OCHF₂, (CF₃)₂CHCF₂OCF₃ und CF₃CF=CFOCH₃.

8. Verfahren zum Herstellen von CF₃CHFCF₂OCF₂H, umfassend die Schritte:
Umsetzen von Methanol mit Hexafluorpropen in Gegenwart einer Base zur Erzeugung von CF₃CHFCF₂OCH₃;
Chlorieren von CF₃CHFCF₂0CH₃ mit Cl₂ zur Erzeugung von CF₃CHFCF₂OCHCl₂; und
Fluorieren von CF₃CHFCF₂OCHCl₂ mit HF zur Erzeugung von CF₃CHFCF₂OCF₂H.

9. Verfahren zum Herstellen eines gesättigten, fluorierten C₄- oder C₅-Hydrofluorether, der ausgewählt ist aus der Gruppe, bestehend aus CF₃CHFCF₂OCH₃, CF₃CHFCF₂OCH₂F, CF₃CHFCF₂OCF₂H, CF₃CHFCF₂OCF₃, (CF₃)₂CHCF₂OCH₃, (CF₃)₂CHCF₂OCH₂F, (CF₃)₂CHCF₂OCHF₂ und (CF₃)₂CHCF₂OCF₃, umfassend die Schritte:
Umsetzen eines C₁-Alkohols mit einem fluorierten C₃- oder C₄-Alken in Gegenwart einer Base zur Erzeugung eines ersten Reaktionsproduktes;
Chlorieren des ersten Reaktionsproduktes mit Cl₂ zur Erzeugung eines zweiten Reaktionsproduktes und
Fluorieren des zweiten Reaktionsproduktes mit HF zur Erzeugung eines gesättigten, fluorierten C₄- oder C₅-Hydrofluorethers.

10. Verfahren nach Anspruch 9, wobei die Base ausgewählt ist aus der Gruppe, bestehend aus Natrium- und Kaliumhydroxid.

11. Zusammensetzung, aufweisend CF₃CHFCF₂OCF₃.

## Revendications

1. Procédé destiné à éteindre un feu comprenant les étapes consistant à introduire dans le feu une concentration éteignant un feu d'une composition comprenant un composé choisi dans le groupe comprenant CF₃CHFCF₂OCH₃, CF₃CHFCF₂OCH₂F, CF₃CHFCF₂OCF₂H, CF₃CHFCF₂OCF₃, (CF₃)₂CHCF₂OCH₃, (CF₃)₂CHCF₂OCH₂F, (CF₃)₂CHCF₂OCHF₂ et (CF₃)₂CHCF₂OCF₃ et à maintenir la concentration du composé jusqu'à extinction du feu.

2. Procédé conforme à la revendication 1, dans lequel le composé est employé à un taux d'au moins 3% (v/v).

3. Procédé conforme à la revendication 1, dans lequel le composé est employé dans un système de noyage total.

4. Procédé conforme à la revendication 1, dans lequel le composé est employé dans un système d'extinction portatif.

5. Procédé conforme à la revendication 1, dans lequel la composition comprend un mélange avec d'autres agents extincteurs de feu.

6. Procédé conforme à la revendication 5 dans lequel les autres agents extincteurs de feu sont choisis dans le groupe comprenant CF₃CHFCF₃, CF₃CF₂CF₂H, CF₃CH₂CF₃, CF₃CF₂H, et CF₃H.

7. Agent extincteur de feu comportant un composé choisi dans le groupe comprenant CF₃CHFCF₂OCH₃, CF₃CHFCF₂OCH₂F, CF₃CHFCF₂OCF₂H, CF₃CHFCF₂OCF₃, (CF₃)₂CHCF₂OCH₃, (CF₃)₂CHCF₂OCH₂F, (CF₃)₂CHCF₂OCHF₂, (CF₃)₂CHCF₂OCF₃ et CF₃CF=CFOCH₃.

8. Procédé de fabrication de CF₃CHFCF₂OCF₂H comprenant les étapes consistant à:
faire réagir du méthanol avec de l'hexafluoropropène en présence de base afin de produire du CF₃CHFCF₂OCH₃;
chlorer le CF₃CHFCF₂OCH₃ avec du Cl₂ afin de produire du CF₃CHFCF₂OCHCl₂; et
fluorer le CF₃CHFCF₂OCHCl₂ avec du HF afin de produire du CF₃CHFCF₂OCF₂H.

9. Procédé de fabrication d'un hydrofluoroéther en C₄ ou C₅ saturé et fluoré choisi dans le groupe comprenant CF₃CHFCF₂OCH₃, CF₃CHFCF₂OCH₂F, CF₃CHFCF₂OCF₂H, CF₃CHFCF₂OCF₃, (CF₃)₂CHCF₂OCH₃, (CF₃)₂CHCF₂OCH₂F, (CF₃)₂CHCF₂OCHF₂ et (CF₃)₂CHCF₂OCF₃, comprenant les étapes consistant à:
faire réagir un alcool en C₁ avec un alcène en C₃ ou C₄ fluoré en présence d'une base afin de former un premier produit de réaction;
chlorer le premier produit de réaction avec du Cl₂ afin de former un second produit de réaction; et
fluorer le second produit de réaction avec du HF afin de former un hydrofluoroéther en C₄ ou C₅ saturé et fluoré.

10. Procédé conforme à la revendication 9, dans lequel la base est choisie dans le groupe comprenant l'hydroxyde de sodium et de potassium.

11. Composition comprenant du CF₃CHFCF₂OCF₃.
